# EUROPEAN PATENT APPLICATION

(11) **EP 4 166 172 A1**
(43) Date of publication of application: **19.04.2023**
(21) Application number: 22200977.1
(22) Date of filing: 11.10.2022
(51) Int. Cl.: A61M 5/31

(54) **SYRINGE COVER DEVICE**

(30) Priority: 13.10.2021 SG 10202111397
(71) Applicant: EPIC MEDICAL PTE LTD, Singapore 069534 (SG)
(72) Inventor: LEE, Eng Hwee Freddie, 069534 Singapore (SG)
(74) Representative: INNOV-GROUP

(57) **Abstract**

A syringe cover device comprises a first cap with a first frame and a first seal coupled thereto, and a second cap with a second frame and a second seal coupled thereto. The first frame and the second frame are couplable to each other to form an accommodating space therebetween. The first seal and the second seal are couplable to each other to form a main seal body and disposed in the accommodating space. The main seal body has a first surface abutting against the first frame and the second frame, a second surface opposite to the first surface and an aperture formed through the first surface and the second surface. The first frame and the second frame may be attached to a syringe flange with the syringe plunger passing through the aperture to cover and seal a back opening of the syringe barrel.

## Description

### Technical Field

The present invention relates to a medical device and in particular, the present invention relates to a device for covering a back opening of a syringe and a syringe incorporating such a device.

### Background

Hazardous drug exposures to presently known syringes in medical applications may lead to serious health complications from skin irritation to birth defects and even carcinogenic, genetic and mutational consequences.

While there are already devices that provide containment of the volatile organic contaminants when the syringe is connected and disconnected, there is an absence of means to control the exposures arising from the contaminated inner surface of the syringe barrel after the contents of the syringe is expelled. The disposal of syringes with its plunger pushed forward and the inner surface of the barrel exposed presents a problematic source of hazardous drug contamination.

### Summary

The present invention provides a solution for reducing exposure of hazardous drugs resulting from its transfer from a vial to a syringe and then from the syringe to an IV infusion bag.

According to one aspect, the present invention provides a syringe cover device for attaching to a syringe that covers a back opening of the syringe and seals the inner volume of the syringe barrel at the back opening of the syringe. According to another aspect, the present invention provides a syringe with a syringe cover device attached to the syringe barrel flange and covers and seals the back end of the syringe barrel. The syringe cover device is to fit and engage to the flange of most commonly available syringes, in either a detachable or non-detachable manner.

Advantages of the invention include to allow the user to decide on the appropriateness of installing the syringe cover device to a standard syringe based on the contents that the syringe would be used to withdraw and expel, unlike the use of expensive syringe with similar or equivalent features. The present invention offers a more cost-effective solution that is appropriate with the actual use situation.

The syringe cover device would be attached to a syringe by the user and disposed off with the syringe after use. In other embodiments, the present invention provides a syringe with a syringe cover device attached thereto, which is provided as a ready to use syringe with a closed barrel cover device.

According to one embodiment, a syringe cover device comprises a first cap with a first frame and a first seal coupled thereto, and a second cap with a second frame and a second seal coupled thereto. The first frame and the second frame are couplable to each other to form an accommodating space therebetween. The first seal and the second seal are couplable to each other to form a main seal body and disposed in the accommodating space. The main seal body has a first surface abutting against the first frame and the second frame, a second surface opposite to the first surface and an aperture formed between the first seal and the second seal and through the first surface and the second surface. The first frame and the second frame may be attached to a syringe flange with the syringe plunger passing through the aperture to cover and seal a back opening of the syringe barrel.

The first frame and the second frame each has a respective first stopper a and a second stopper extending towards the accommodating space, the first surface of the main seal body is positioned to abut against the first stopper and the second stopper. Upon the first frame and the second frame being secured to a syringe, the first seal and the second seal clamp a plunger strut of the syringe therebetween such that the plunger strut movably passes through the aperture, and the second surface of the main seal body is brought into abutment against a flange of the syringe such that the first seal and the second seal cover a back opening of the syringe.

The aperture has four slits evenly distributed in a polar array about a longitudinal axis a, each slit extending in a radial direction perpendicular to the longitudinal axis, the first frame having a first retaining arm coupled thereto and the second frame having a second retaining arm coupled thereto. The first retaining arm and the second remaining arm are distributed symmetrical about the longitudinal axis and spaced apart from each other with an arm distance therebetween greater than a first width of the flange.

The first frame and the second frame are attachable to the flange and positioned between a first annular position and a second annular position. Upon being at the first annular position, the first retaining arm abuts against a first finger-holding portion a of the flange and the second retaining arm abuts against an opposite second finger-holding portion of the flange and upon being at the second annular position, the first retaining arm abuts against the second finger-holding portion of the flange and the second retaining arm abuts against the first finger-holding portion of the flange.

The main seal body has a first fin formed on the first surface and at a periphery of the aperture, and a second fin formed on the second surface and at a periphery of the aperture. Upon the first frame and the second frame being secured to the syringe, the first fin and the second fin bias against the plunger strut to form a plunger sealing interface.

The main seal body has an annular ridge to abut against the flange of the syringe to form a flange sealing interface. The plunger sealing interface and the flange sealing interface seal the back opening of the syringe to prevent liquid communication therethrough. The flange sealing interface and the second flange sealing interface may be oriented orthogonal to each other.

The first seal and the second seal each has a pair of radial recesses, and the first frame and the second frame each has a pair of radial projections, each of which being received in a respective radial recess.

The syringe cover device further comprises an orifice passing through the first frame and an air filter covering the orifice . The orifice establishes air communication between an inner space of the barrel and an outer space of the barrel and through the air filter.

The syringe cover device further comprises a first support arm extending radially from the first frame and a second support arm extending radially from the second frame, a hinge pin projecting axially from the first support arm, and a hinge bearing formed on the second support arm. The hinge pin is rotatably received in the hinge bearing to enable relative movement between the first frame and the second frame relative to each other. The hinge bearing includes an elongated and curved groove. The hinge pin is rotatable and slidable relative to the hinge bearing about the groove to vary a positional relationship between the first frame and the second frame.

The syringe cover device further comprises a lock arm coupled to the first frame and a lock catch coupled to the second frame. Upon the first frame and the second frame being coupled to each other the lock arm is engaged with the lock catch to secure the first frame and the second frame to each other.

According to another aspect, the present invention provides a syringe which comprises a barrel and a plunger movably disposed in the barrel, and a cover device coupled to the barrel and the plunger. The barrel has a front opening, a back opening and a flange formed at the back opening. The plunger has a plunger strut passing through the back opening and the flange. The cover device caps the back opening of the barrel. The cover device has an aperture through which the plunger strut movably passes, and an end surface abutting against the flange of the barrel. The aperture and the plunger strut form a first seal interface to the back opening, and the end surface of the cap and the flange form a second seal interface to the back opening.

The cover device may comprise one or more or up to all the features according to the syringe cover device of the previous embodiment.

### Brief description of drawings

The features of the embodiments will be more comprehensively understood in conjunction with the accompanying drawings, in which:
Fig. 1 is a perspective top view of a syringe cover device according to one embodiment of the present invention;
Fig. 2 is a perspective bottom view of Fig. 1;
Fig. 3 is a partial exploded view of Fig. 1;
Fig. 4 is a perspective top view showing the syringe cover device of Fig. 1 at disassembled status;
Fig. 5 is a perspective bottom view of Fig. 4;
Fig. 6 is a perspective view showing the syringe cover device of Fig. 1 when attached to a syringe;
Fig. 7 is an enlarged view of portion 7 of Fig. 6;
Fig. 8 is a partial exploded view of Fig. 6;
Fig. 9 is a side view of Fig. 6;
Fig. 10 is a cross-sectional bottom view of Fig. 9 along A-A;
Fig. 11 is a partial cross sectional view of Fig. 6 along B-B;
Fig. 12 is a partial cross sectional view of Fig. 6 along C-C;
Fig. 13 is an enlarged view of portion 13 of Fig. 11;
Fig. 14 is an enlarged view of portion 14 of Fig. 12;
Fig. 15 is a partial exploded view of Fig. 12;
Fig. 16 is a partial enlarged view of portion 16 of Fig. 15;
Fig. 17 is an enlarged side view of portion 16 of Fig. 15;

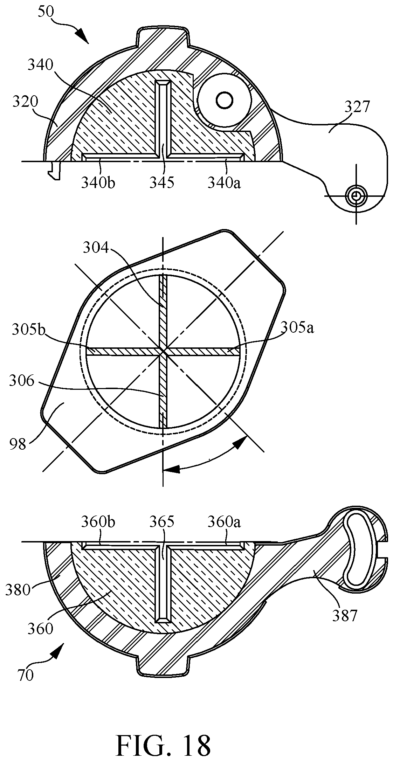

Fig. 18 is a partial exploded top view of Fig. 6;
Figs. 19A, 19B, 19C and 19D are perspective views showing a method of attaching a syringe cover device of Fig. 1 to a syringe;

### Detailed description of the invention

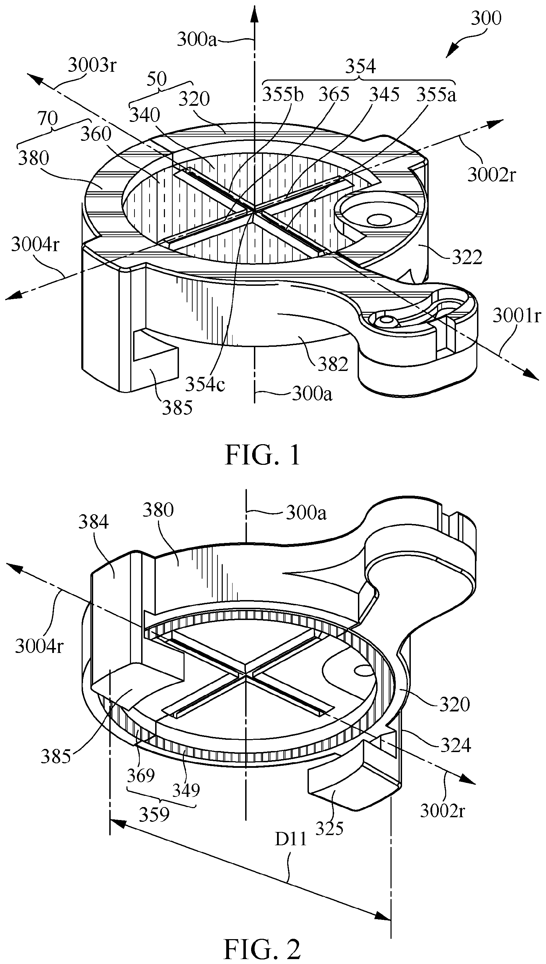

As shown in Fig. 1 to Fig. 5, according to one embodiment, a syringe cover device 300 includes a first cap 50 and a second cap 70, in the form of for example a first semi disc and a second semi disc of a complementary structure, respectively. The first cap 50 includes a first frame 320 of a semicircle shape and a first seal 340 partially surrounded by and coupled to a concave side of the first frame 320. The second semi disc includes a second frame 380 of a semicircle shape and a second seal 360 partially surrounded by and coupled to a concave side of the second frame 380. The first cap 50 and the second cap 70 are movable relative to each other in its main plane around its hinge-like joint which is formed by a hinge pin 328 formed on the first frame 320, and a hinge bearing 388 formed on the second frame 380. When the first cap 50 and the second cap 70 are positioned with its diameter adjacent and aligned to each other, the resultant structure is a full-disc-shaped cover device which approximately corresponds in size and shape to an open back end of a syringe barrel 90.

In the embodiment as illustrated therein, the first frame 320 and the second frame 380 are couplable to each other, to form an accommodating space 350 between the first frame 320 and the second frame 380. The first and second seals 340, 360 are couplable to each other to form a main seal body 352, and the main seal body 352 is disposed in the accommodating space 350 surrounded by the first and second frames 320, 380. The main seal body 352 has a first surface 352a, a second surface 352b opposite to the first surface and an aperture 354. The aperture 354 is formed between the first seal 340 and the second seal 360 and through the first surface 352a and the second surface 352b.

The first frame 320 has a first stopper 320a. The second frame 380 has a second stopper 380a. The first stopper 320a and the second stopper 380a are located at or adjacent to the first surface 352a, and extend towards the accommodating space 350. The first surface 352a of the main seal body 352 abuts against the first stopper 320a and the second stopper 380.

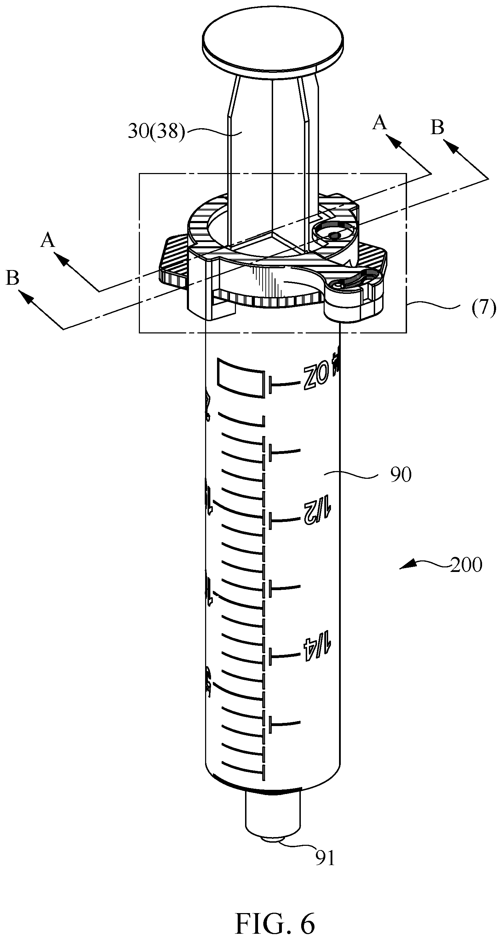

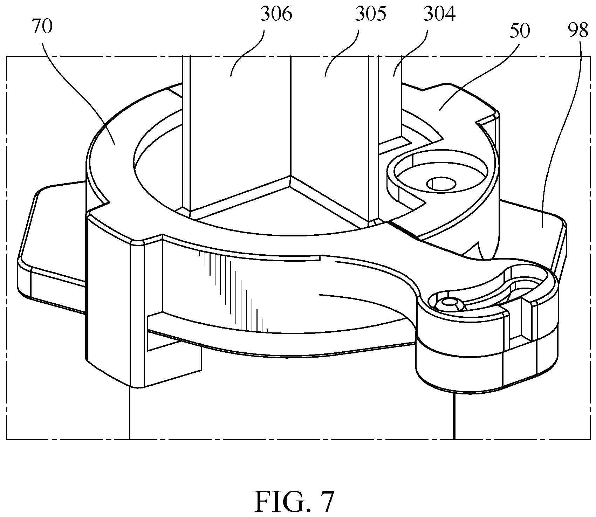

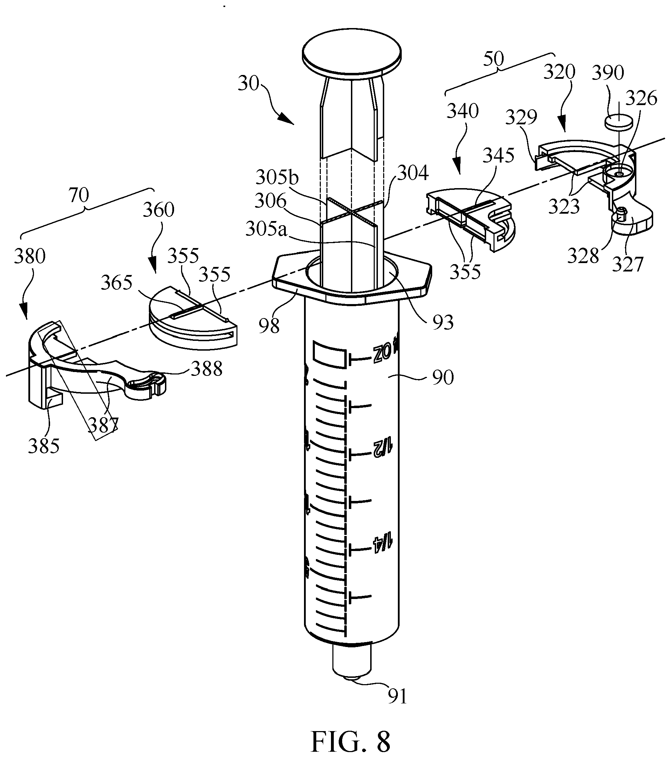

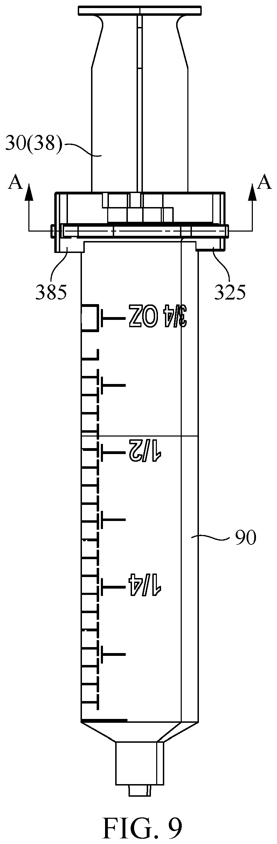

As shown in Fig. 6 to Fig. 10, a syringe 200 includes a barrel 90 and a plunger 30 movably disposed in the barrel 90. The barrel 90 has a front opening 91, a back opening 93 and a flange 98 formed at the back opening 93. The plunder 30 has a plunger strut 38 passing through the back opening 93 and the flange 98. The first seal 340 and the second seal 360, made of e.g. soft pliable material, enclosed within the first and second frames 320, 380 act as seals 340, 360 that encloses the volume space formed by the inner walls of the barrel 90 and the strut 38 of the plunger 30.

Upon the first frame 320 and the second frame 380 being secured to syringe 200, the first seal 340 and the second seal 360 clamp the plunger strut 38 of the syringe 200 therebetween such that the plunger strut 38 movably passes through the aperture 354. The second surface 352b of the main seal body 352 is brought into abutment against the flange 98 of the syringe barrel 90. With the first frame 320, the second frame 380, the first seal 340 and the second seal 360 coupled to the syringe 200 in the above-illustrated manner, the first seal 340 and the second seal 360 form the main seal body 352 to cover the back opening 93 of the syringe 200.

The first and second seals 340, 360 are formed by any soft, pliable material suitable for use as air-tight and liquid-tight seals for a syringe. For example, the first and second seals 340, 360 are formed of thermoplastic elastomer, silicone, rubber, isoprene or the like. The first frame 320 and the second frame 380 are formed of a material suitable to provide necessary structural strength to support and secure the first and second seals 340, 360 to the syringe 200. The first frame 320 and the second frame 380 are formed of materials with structural strength and rigidity higher than the material of the first and second seals 340, 360. The first seal 340, second seal 360, first frame 320 and second frame 380 may be formed individually by e.g. over mold injection process or 3D printing, and assembled together by fitting and/or coupling the first seal 340 to the concave side of the first frame 320 to form the first cap 50 which is an assembled component, and by fitting and/or coupling the second seal 360 to the concave side of the second frame 380 to form the second cap 70 which is also an assembled component. Alternatively, the rigid first frame 320 and second frame 380 are firstly made of a first material by e.g. inject molding, and followed by injecting a second material that fills the area defined by the first seal 340 and the second seal 360. The firstly formed first and second frames 320, 380 retain the soft, pliable material that forms the first and second seals 340, 360, to form the integral first cap 50 and second cap 70, respectively. Accordingly. the first seal 340 may be formed to the concave side with the first frame 320 by insert-molding process, such that the first seal 340 and the first frame 320 form the first cap 50 which is an integrally structured component. Likewise, the second seal 360 may be formed to the concave side with the second frame 380 by insert-molding process, such that the second seal 360 and the second frame 380 form the second cap 70 which is also an integrally structured component.

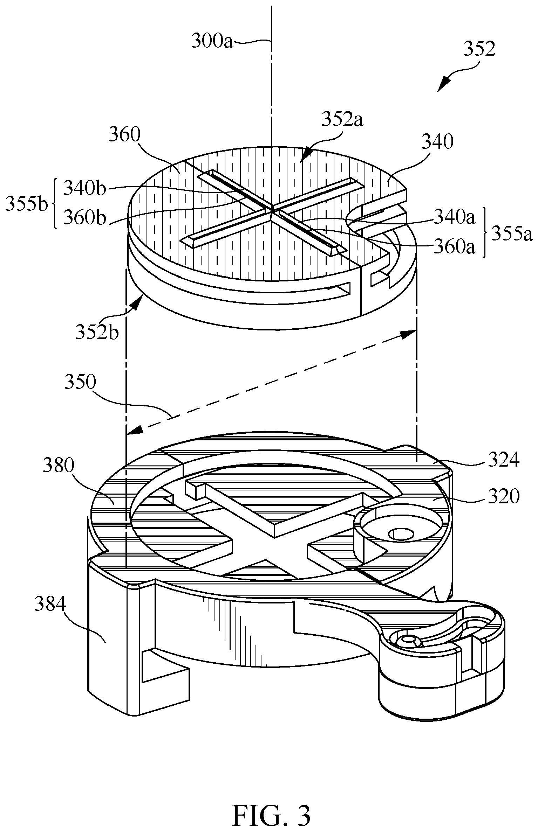

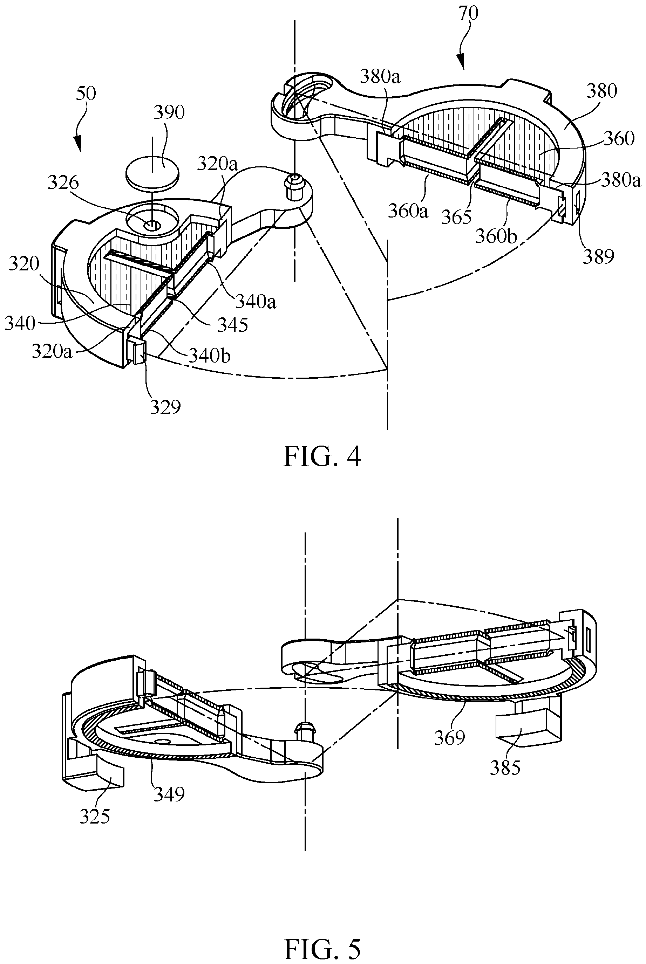

As shown in Fig. 3 and Fig. 4, the first seal 340 has a first slit 345 and a pair of first recesses 340a, 340b formed thereon. The first slit 345 and each of the pair of first recesses 340a, 340b have a same length, and each of the pair of first recesses 340a, 340b has a width which is half of the width of the first slit 345. Likewise, the second seal 360 has a second slit 365 and a pair of second recesses 360a, 360b formed thereon. The second slit 365 and each of the pair of second recesses 360a, 360b have a same length as the first slit 345 and the pair of first recesses 340a, 340b. The second slit 365 has the same width as the first slit 345. Each of the pair of second recesses 360a, 360b has a width which is half the width of the second slit 365.

The first seal 340 and the second seal 360 form the main seal body 352 and with the aperture 354 formed between the first seal 340 and the second seal 360 and through the main seal body 352. The aperture 352 therefore has four aperture slits 345, 355a, 355b and 365, in which, aperture slit 355a is formed by one of the pair of first recess i.e. first recess 340a and one of the pair of second recess i.e. second recess 360a joining the first recess 340a. The aperture slit 355b is formed by the other one of the pair of first recesses, i.e. first recess 340b and the other one of the second recesses i.e. second recess 360b joining the first recess 340b. Accordingly, the aperture 354 has four slits / recesses 345, 355a, 355b and 365 formed on respective first and second seals 340, 360 which join to each other to form the main seal body 352.

As shown in Fig. 1, Fig. 2, Fig. 3 and Fig. 10, the four slits 345, 355a, 355b, 365 are evenly distributed in a polar array about a longitudinal axis 300a passing through a geometric center 354c of the aperture 354. Each slit 345, 355a, 355b, 365 extends in a respective radial direction 3001r, 3002r, 3003r and 3004r perpendicular to the longitudinal axis 300a. The first frame 320 has a first retaining arm 324 coupled thereto. The second frame 380 has a second retaining arm 384 coupled thereto. The first retaining arm 324 and the second remaining arm 384 are positioned spaced apart from each other in opposite radial directions and symmetrical with respect to the longitudinal axis 300a, for example in respective second radial direction 3002r and 3004r, and with an arm distance D11 between the first retaining arm 324 and the second retaining arm 384 greater than a first width W11 of the flange 98.

The first frame 320 and second frame 340 are attachable to the flange 98 between a first annular position P11 and a second annular position P12 which is 90-degree apart from the first annular position P11 with respect to the longitudinal axis 300a. At the first position P11, the first retaining arm 324 abuts against a first finger-holding portion 98a of the flange 98 and the second retaining arm 384 abuts against a second finger-holding portion 98b of the flange 98 positioned opposite to the first finger-hold portion 98a. At the second position P12, the first retaining arm 324 abuts against the second finger-holding 98b portion of the flange 98, and the second retaining arm 384 abuts against the first finger-holding portion 98a of the flange 98.

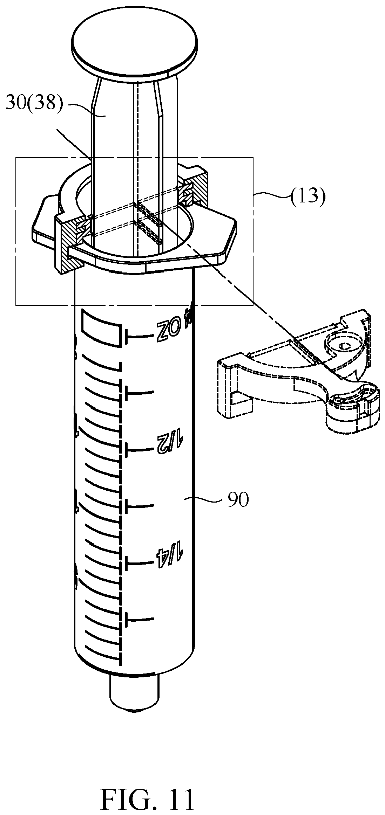

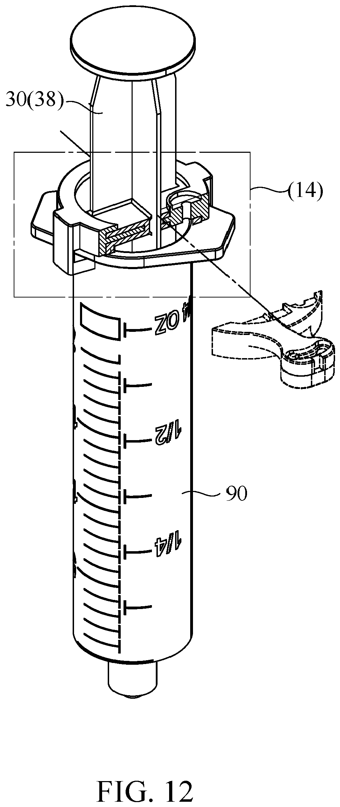

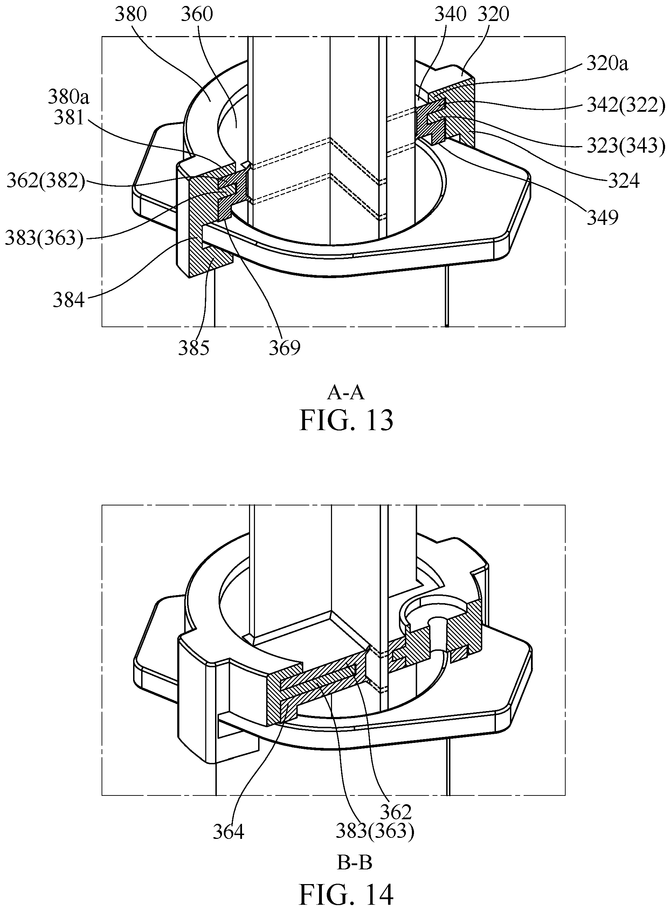

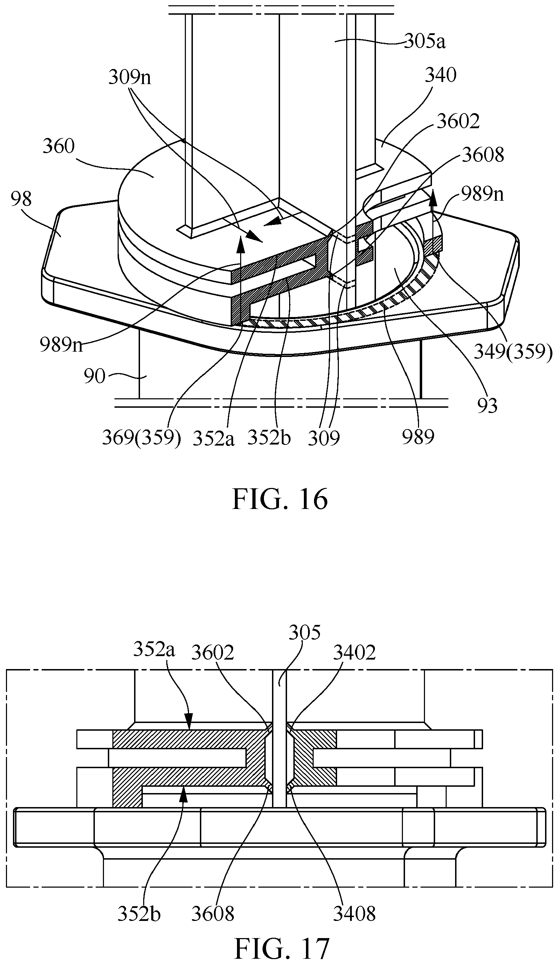

As shown in Fig. 11 to Fig. 17, the main seal body 352 has a first fin 3402, 3602 formed on the first surface 352a and at a periphery of the aperture 354, and a second fin 3408, 3608 formed on the second surface 352b and at a periphery of the aperture 354. Upon the first frame 320 and the second frame 380 being secured to the syringe 200, the first fin 3402, 3602 and the second fin 3408, 3608 bias against the vanes of the plunger strut 38, e.g. vane 305a as shown in Fig. 16, to form a plunger sealing interface 309.

The main seal body 352 has an annular ridge 359 formed by the first half ridge 349 of the first seal 340 and the second half ridge 369 of the second seal 360. The annular ridge 359 abuts against the flange 98 of the syringe 200 to form a flange sealing interface 989. Upon the device 300 being coupled to the flange 98 of the syringe 200, the first seal 340 and second seal 360 together with the plunger sealing interface 309 and the flange sealing interface 989 prevent liquid communication between an inner space and an outer space of the barrel 90 through the back opening 93. As such, any liquid leakage from the inner space of the syringe to the outer space is prevented.

The plunger sealing interface 309 and the flange sealing interface 989 are oriented orthogonal to each other, as shown in Fig. 16 by respective normal direction 309n of the plunger sealing interface 309 and 989n of the flange sealing interface 989.

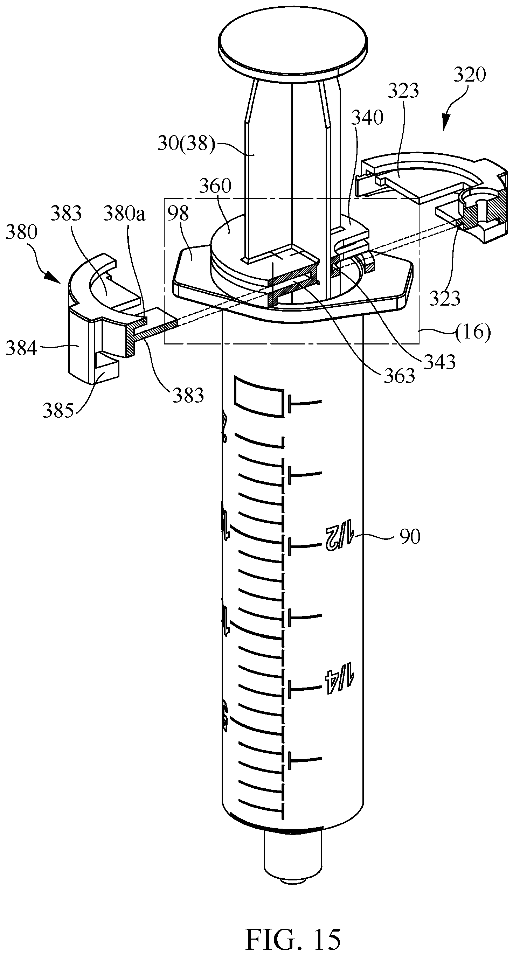

As shown in Fig. 15, the first seal 340 has a pair of first radial recesses 343. The second seal 360 has a pair of second radial recesses 363. The first frame 320 has a pair of first radial projections 323, and the second frame 380 has a pair of second radial projections 383. The pair of first radial projections 323 are received and embedded in the pair of first radial recesses 343. The pair of second radial projections 383 are received and embedded in the pair of second radial recess 363. Positioned in the above-illustrated manner, the first and second frames 320, 380 provide stable and reliable structural support at the inner portion of the first seal 340 and the second seal 360, to secure and maintain the first and second seals 340 and 360 to the syringe flange 98 and plunger strut 38.

The cover device 300 may include an orifice 326 formed on the first frame 320. The orifice 326 establishes air communication between an inner space of the barrel 90 and an outer space of the barrel 90, to ease the operation of pushing / pulling of the plunger 30 into / from the syringe barrel 90. The cover device 300 may further include a filter 390 covering the orifice 326. Filter 390 may be made of materials that allows air to pass through but prevent liquid and solid contents from passing through. Filter 390 prevents contaminants from leaking through the orifice 326 or entering the barrel 90 via the back opening 93 during operation of the syringe 200 when the plunger 30 is pulled or pushed.

The syringe cover device 300 may include a hinge pin 328 projecting axially from the first frame 320 and a hinge bearing 388 formed in the second frame 380. The hinge pin 328 is rotatably and slidably received in the hinge bearing 388 to form a hinge 358. Hinge 358 allows relative movement between the first frame 320 and the second frame 380 about the hinge 358. The hinge bearing 388 includes an elongated and curved groove 388a. The hinge pin 328 is rotatable in the groove 388a and independently or concurrently, the hinge pin 328 is slidable along the groove 388a to, vary a positional relationship between the first frame 320 and the second frame 380.

The syringe cover device 300 may include a lock arm 329 coupled to or formed integral with the first frame 320, and a lock catch 389 coupled to or formed integral with the second frame 380. The lock arm 329 and the lock catch 389 are positioned opposite to the hinge 358 respect to the center portion of the syringe cover device 300. Upon the first frame 320 and the second frame 380 being coupled to each other and with the syringe flange 90 clamped between the first seal 340 and the second seal 360, the hinge pin 328 is engaged with the hinge bearing 388 and the lock arm 329 is engaged with the lock catch 389, to lock the first frame 320 and the second frame 380 to each other and secure to the first seal 340 and the second seal 360 to cover and seal the back opening 93 of the barrel 90 of the syringe 200.

The aperture 354 has a perimeter rim matching the cross-sectional profile of a syringe plunger strut 38. Upon the first cap 50 and the second cap 70 being attached to a syringe 200, by e.g. the first frame 320 and second frame 380 clamping the strut 38 of the syringe plunger 30 therebetween, the syringe plunger strut 38 is disposed to pass through the aperture 354, and the rim of the aperture 354 is brought into contact with the strut 38 of the plunger 30, with the fins 3402, 3602, 3408 and 3608 elastically biasing against the struct 38. Engagement of the first seal 340 and the second seal 360 with the fins 3402, 3602, 3408 and 3608 elastically biasing against the strut 38 allows the movement of the syringe plunger 30 relative to the barrel 90 without significant obstruction or resistance, i.e. the frictional force between the slits 345, 355 and 365 of the aperture 354 and the strut 38 of the syringe plunger 30 is negligible from the point of view of a user operating the syringe 200. In the meantime, the engagement between the fins 3402, 3602, 3408 and 3608 and the struct 38 forms a liquid-tight seal to prevent leakage of liquid contents from the barrel 90.

The slits 345, 355 and 365 of the seals 340, 360 are formed in a manner which, upon the first seal 340 and the second seal 360 being coupled to each other, form the aperture 354 that accommodate and adapt to the cross-sectional profile and structure of the syringe plunger 30. In particular, the strut 38 and vanes 304, 305a, 305b and 306 structured to reinforce the plunger 30 in pushing / pulling the contents drawn into / from the syringe barrel 90, are received and in contact with the perimeter rim of the aperture 354 via the fins 3402, 3602, 3408 and 3608 elastically.

In use, when a standard syringe is presented to the user, the cross formed at the intersectional of the vanes 304, 305a, 305b and 306 could be in any arbitrary orientation relative to the open end of the syringe barrel 90. The cover device 300 could be easily attached to the open back end 93 of the barrel 90 without the need to manually vary or reorientate the angular position of the strut 38 and hence the vanes 304, 305a, 305b and 306 of the plunger 30 along the annular direction of the plunger 30 relative to the barrel 90.

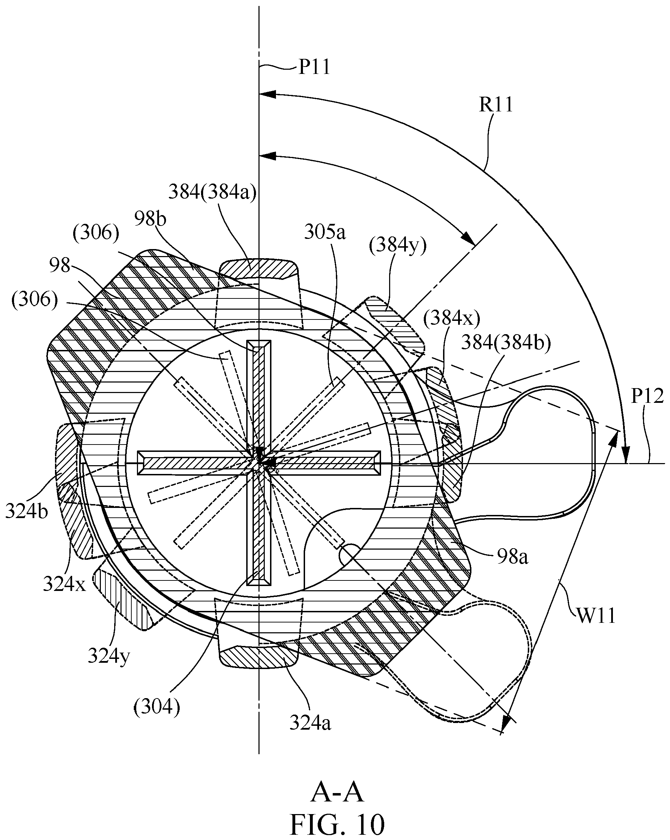

As shown in Fig. 10 with further details, a first annular position P11 and a second annular position P12 define an annular range R11 of an approximately 90-degree angle within which at least one of the vanes 304, 305a, 305b and 306 of the plunger strut 38 is disposed. The situation where two of the vanes e.g. vanes 304 and 306 are disposed at position P1 represents the extreme position at which all the four vanes are angularly oriented respect to the flange 90. When vanes 304 and 306 are disposed at position P1, the first frame 320 and the second frame 380 are able to be clamped to the flange 90 by the first arm 324 and second arm 384 abutting against the respect first and second hand-holding portions 98a and 98b. In the situation where one of the vanes e.g. vane 306 is positioned beyond position P1 i.e. access of vane 306 is hindered by the second hand-holding portion 98b, there will be a neighboring vane i.e. vane 305a disposed within the annular range R11 and which is therefore accessible for being received by a slit of one of the first seal 340 and second seal 360, as a start of attaching the syringe cover device to the syringe.

As such., the syringe cover device 300 could be attached to the open end 93 of the barrel 90 of any syringe, regardless of the orientation of the vanes 304, 305a, 305b and 306 on the syringe plunger 30. This is achieved by locating the hinge pin 328 and the hinge bearing 388 that connects the first cap 50 and second cap 70 at a position further away from the edge of the syringe flange 98, so that the circular movement traced by the arms 327, 387 extended from the hinge pin 328 and hinge groove 388 is not obstructed by any part of the syringe. This feature obviates the need to manually align the annular orientation of the syringe plunger 30, such that the slits 345, 355a, 355b and 365 of the first and second seals 340, 360 of the first and second caps 50, 70 could be affixed around the vanes of the plunger 30 and allow the first and second caps 50, 70 to form a complete round disc-shaped cover without being obstructed by the syringe flange 98. In the situation where two of the four diametrically located vanes of the plunger 30 are aligned with and obstructed by the finger-holding portions 98a, 98b of the syringe flange 98 from inserting into the slits of the first seal 340 and second seal 360, the other two perpendicularly positioned vanes will become accessible from the sides of the flange 98 where no finger-hold 98a, 98b is in place, hence such other two vanes can be aligned and inserted into the slits of the first seal 340 and the second seal 360.

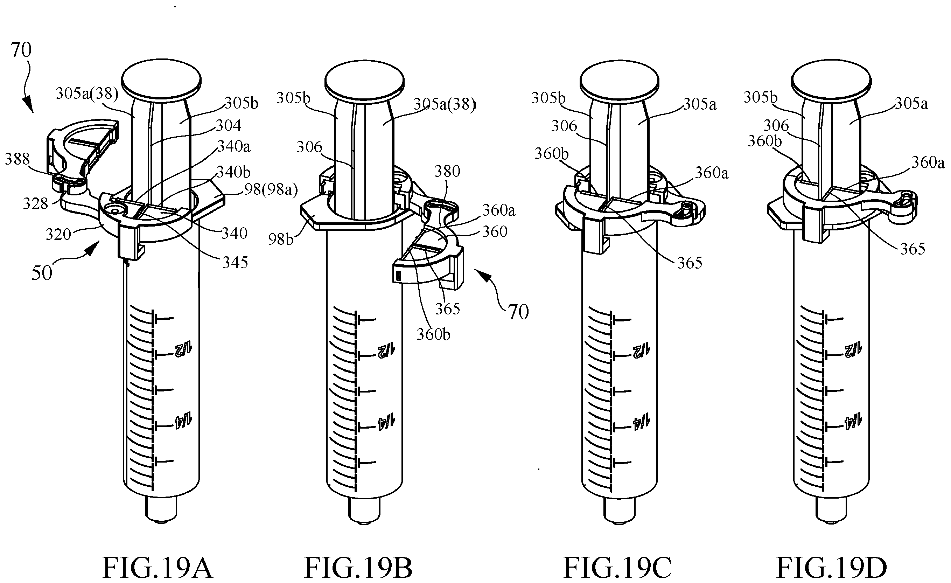

To attach the cover device 300 to the open end of the syringe barrel 90, as shown in Figs. 19A to 19D, first cap 50 and second cap 70 are firstly separated by pivoting away from each other about the hinge pin 328 and hinge bearing 388, to expose the slits 345, 340a, 340b, 365 360a and 360b. The second seal 70 further moves away from the first seal 50 by the hinge pin 328 sliding in the hinge bearing 388 , as shown in Fig. 19A, one slit of the soft pliable material seal located in one of the seals 340, 360, e.g. the slit 345 of the first seal 340 disposed in the first frame 320, is slide-fit and affixed around one of the vanes of the syringe plunger strut 38, e.g. vane 304 which is positioned facing away from the finger-hold portions 98a and 98b of the flange 98. In the meantime, the neighboring vanes 305a, 305b of the plunger strut 38 are aligned with and fit into the recesses 340a, 340b of the first seal 340.

After the first seal 340 is positioned with the vane 305 fully received in the slit 345, as shown in Fig. 19B and Fig. 19C, the second seal 70 is rotated towards the strut 38 and with the opposite slit 365 aligned with the corresponding opposite slit 306. Further, as shown in Fig. 19D, the second seal 70 is pushed towards the strut 38 by the hinge pin 328 sliding along the hinge bearing 388, until the vane 306 is fully revised in the slit 365. In the meantime, the other two vanes 305a 305b are clamped between the first and slits 340a, 340b and 360a, 360b, respectively. Upon moving the second frame 380 around the hinge pin 328 along the hinge groove 388 such that the first and second frames 320 and 380 are coupled to each other, the area between all four vanes 304, 305a, 305b and 306 of the plunger 30 that form four distinct quadrants become sealed by the slits 345, 355a, 355b and 365 of first and second seals 340, 360. The tolerances of the openings / seams between the slits 345, 355a, 355b and 365 and the struts 38 of the plunger 30 allow the volume space within the inner walls of the barrel 90 and the plunger 30 to be liquid-tight and isolated from the outside environment, even when the plunger 30 travels longitudinally along the axis of the barrel 90. The volume space between the back opening 93 of the barrel 90 and the piston of the syringe decreases when the plunger30 is pulled as in drawing fluid contents into the syringe 200, and correspondingly increases when the plunger30 is pushed as in expelling the fluid contents out of the syringe 200. The first seal 340 and second seal 360 are secured to the flange 90 by a lock arm or hook 329 formed on the first frame 320 and a lock catch or pocket 389 formed on the second frame 380, to retain the device in its place at the open end of the barrel 90, for example by clamping the catch members 325, 385 formed on the respective first frame 320 and the second frame 380 onto the flange 98 of the barrel 90 .

As used herein, the article in singular form "a" and "an" may be construed as including the plural meaning of "one or more" unless clearly indicated otherwise.

This disclosure has been presented for purposes of illustration and description but is not intended to be exhaustive or limiting. Many modifications and variations will be apparent to those of ordinary skill in the art. The example embodiments were chosen and described in order to explain principles and practical application, and to enable others of ordinary skill in the art to understand the disclosure for various embodiments with various modifications as are suited to the particular use contemplated.

Thus, although illustrative example embodiments have been described herein with reference to the accompanying figures, it is to be understood that this description is not limiting and that various other changes and modifications may be affected therein by one skilled in the art without departing from the scope of the disclosure as defined by the claims appended hereto.

## Claims

1. A syringe cover device comprising:
a first frame and a first seal coupled to the first frame;
a second frame and a second seal coupled to the second frame;
the first frame and the second frame being couplable to each other to surround the first seal and the second seal therebetween;
the first seal and the second seal being couplable to each other to form a main seal body and disposed between the first frame and the second frame, the main seal body having a first surface abutting against the first frame and the second frame, a second surface opposite to the first surface and an aperture formed between the first seal and the second seal and through the first surface and the second surface.

2. The syringe cover device of claim 1,
wherein the first frame and the second frame each having a respective first stopper and a second stopper extending towards the respective first seal and the second seal, the first surface of the main seal body being positioned to abut against the first stopper and the second stopper, and
wherein upon the first frame and the second frame being secured to a syringe, the first seal and the second seal clamp a plunger strut of the syringe therebetween such that the plunger strut movably passes through the aperture, and the second surface of the main seal body is brought into abutment against a flange of the syringe such that the first seal and the second seal cover a back opening of the syringe.

3. The syringe cover device of claim 2, wherein the aperture has four slits evenly distributed in a polar array about a longitudinal axis passing through a geometric center of the aperture, each slit extending in a radial direction perpendicular to the longitudinal axis, the first frame having a first retaining arm and the second frame having a second retaining arm, wherein the first retaining arm and the second remaining arm are positioned symmetrical with respect to each other about the longitudinal axis and spaced apart from each other with an arm distance therebetween, wherein the arm distance is greater than a first width of the flange.

4. The syringe cover device of claim 3, wherein the first frame and the second frame are engageable to the flange and positioned between a first annular position and a second annular position about the longitudinal axis, wherein upon being at the first annular position, the first retaining arm abuts against a first finger-holding portion of the flange and the second retaining arm abuts against an opposite second finger-holding portion of the flange, and upon being at the second annular position, the first retaining arm abuts against the second finger-holding portion of the flange, and the second retaining arm abuts against the first finger-holding portion of the flange.

5. The syringe cover device of claim 2, wherein the main seal body has a first fin formed on the first surface and at a periphery of the aperture, and a second fin formed on the second surface and at a periphery of the aperture, wherein upon the first frame and the second frame being secured to the syringe, the first fin and the second fin bias against the plunger strut to form a plunger sealing interface, wherein the main seal body having an annular ridge to abut against the flange of the syringe to form a flange sealing interface, wherein the plunger sealing interface and the flange sealing interface seal a back opening of the syringe to prevent liquid communication therethrough, and wherein the flange sealing interface and the second flange sealing interface are oriented orthogonal to each other.

6. The syringe cover device of claim 2, wherein the first seal and the second seal each has a pair of radial recesses and the first frame and the second frame each has a pair of radial projections each of which being received in a respective radial recess.

7. The syringe cover device of claim 2 further comprising an orifice passing through the first frame and an air filter covering the orifice, wherein the orifice establishes air communication through the air filter between an inner space of the barrel and an outer space of the barrel.

8. The syringe cover device of claim 2 further comprising a first support arm extending radially from the first frame and a second support arm extending radially from the second frame, a hinge pin projecting axially from the first support arm and a hinge bearing formed on the second support arm, wherein the hinge pin is rotatably and slidably received in the hinge bearing to enable relative movement between the first frame and the second frame relative to each other.

9. The syringe cover device of claim 8, wherein the hinge bearing includes an elongated groove about which the hinge pin is rotatable and along which the hinge pin is slidable to vary a positional relationship between the first frame and the second frame.

10. The syringe cover device of claim 1, further comprising a lock arm coupled to the first frame and a lock catch coupled to the second frame, wherein upon the first frame and the second frame being coupled to each other the lock arm is engaged with the lock catch to secure the first frame and the second frame to each other.

11. A syringe comprising:
a barrel having a front opening, a back opening and a flange formed at the back opening;
a plunger having a plunger strut movably disposed through the back opening and the flange;
a first seal and a second seal coupled to each other to form an aperture through which the plunger strut passes;
a first frame and a second frame coupled to each other to surround the first seal and the second seal therebetween and to clamp the first seal and the second seal to the flange.

12. The syringe of claim 11,
wherein the first frame and the second frame are locked to each other to maintain contact of the first seal and the second seal against the plunger strut, and
wherein the first frame and the second frame are secured to the flange to maintain abutment of the first seal and the second seal against the flange.

13. The syringe of claim 12,
wherein the first frame and the second frame each having a respective first stopper and a second stopper extending towards the plunger strut, the first seal and the second seal being positioned between the respective first stopper and the second stopper and the flange.

14. The syringe of claim 11, wherein the aperture has four slits evenly distributed in a polar array about a longitudinal axis of the syringe, each slit extending in a radial direction perpendicular to the longitudinal axis, the plunger strut having four vanes oriented parallel to the longitudinal direction, each vane being received in a corresponding one of the slits, the first frame having a first retaining arm coupled thereto and the second frame having a second retaining arm coupled thereto, wherein the first retaining arm and the second remaining arm are positioned symmetrical about the longitudinal axis and spaced apart from each other with an arm distance therebetween greater than a first width of the flange.

15. The syringe of claim 14, wherein the first frame and the second frame are rotatable relative to the flange about the longitudinal axis between a first annular position and a second annular position; wherein upon the first frame and the second frame being at the first annular position, the first retaining arm abuts against a first finger-holding portion of the flange and the second retaining arm abuts against a second finger-holding portion of the flange; and wherein upon the first frame and the second frame being at the second annular position, the first retaining arm abuts against the second finger-holding portion of the flange and the second retaining arm abuts against the first finger-holding portion of the flange.

16. The syringe of claim 11, further comprising a first fin and a second fin at a rim of the aperture and extending towards and biasing against the plunger strut to form a plunger sealing interface, the first fin being formed on a first surface of the first seal and the second seal, the second fin being formed on a second surface of the first seal and the second seal,
wherein the first seal and the second seal form an annular ridge a the second surface to abut against the flange to form a flange sealing interface, wherein the plunger sealing interface and the flange sealing interface seal the back opening of the syringe to prevent liquid communication therethrough, and wherein the flange sealing interface and the second flange sealing interface are oriented orthogonal to each other.

17. The syringe of claim 11, wherein the first seal and the second seal each has a pair of radial recesses and the first frame and the second frame each has a pair of radial projections each of which being received in a respective radial recess.

18. The syringe of claim 11, further comprising an orifice passing through the first frame and an air filter 390 covering the orifice, the orifice establishes air communication through the air filter between an inner space of the barrel and an outer space of the barrel.

19. The syringe of claim 11 further comprising a first support arm extending radially from the first frame and a second support arm extending radially from the second frame, a hinge pin projecting axially from the first support arm and a hinge bearing formed on the second support arm, wherein the hinge pin is rotatably received in the hinge bearing to enable relative movement between the first frame and the second frame relative to each other.

20. The syringe of claim 19, wherein the hinge bearing is an elongated groove about which the hinge pin is rotatable and along which the hinge pin is slidable to vary a positional relationship between the first frame and the second frame.
